# EUROPEAN PATENT SPECIFICATION

(11) **EP 1 370 150 B1**
(45) Date of publication and mention of the grant of the patent: **21.11.2007**
(21) Application number: 02716644.6
(22) Date of filing: 25.03.2002
(51) Int. Cl.: A23G 4/00

(54) **ONE-STEP PROCESS FOR PREPARING CHEWING GUM**
EINSTUFIGES VERFAHREN ZUR HERSTELLUNG VON KAUGUMMI
PROCEDE DE PREPARATION DU CHEWING-GUM EN UNE ETAPE

(30) Priority: 23.03.2001 DK 200100492; 06.07.2001 US 303135 P
(43) Date of publication of application: 17.12.2003
(73) Proprietor: Gumlink A/S, 7100 Vejle (DK)
(72) Inventor: WITTORFF, Helle, DK-7120 Vejle (DK); ANDERSEN, Lone, DK-5500 Middelfart (DK); ISAKSEN, Anette, DK-6000 Kolding (DK)
(74) Representative: Olesen, Kaj
(86) International application number: PCT/DK2002/000202
(87) International publication number: WO 2002/076229

(56) References cited:
- EP-A- 1 066 759
- WO-A-99/27798
- US-A- 3 440 060
- US-A- 4 329 369
- US-A- 4 968 511
- US-A- 5 672 367
- US-A- 5 976 581
- US-A- 6 017 565

## Description

### FIELD OF THE INVENTION

The present invention pertains to the field of manufacturing chewing gum. In particular, there is provided a novel one-step manufacturing process, which is generally applicable for chewing gum formulations. In specific embodiments the present invention provides a method of producing chewing gum containing in the gum base part a degradable or biodegradable polymer.

### TECHNICAL BACKGROUND AND PRIOR ART

For the most part, current conventional processes for manufacturing chewing gum, including bubble gums, comprise at least two separate steps. In the first process step, a chewing gum base, typically comprising a variety of elastomeric and resinous compounds, is made which, in a second subsequent step, is compounded with various combinations of chewing gum additives such as bulk sweeteners, colouring agents, flavouring agents and other ingredients to obtain the final chewing gum. Generally, the pre-compounded gum base is softened by heating at a temperature in the range of 100 to 150°C prior to being admixed with the chewing gum additives.

Conventional chewing gum bases are generally prepared on an industrial scale by heating and mixing the various ingredients such as elastomers, resins, inorganic fillers, waxes, fats, emulsifiers, etc. in an appropriate mixing apparatus such as an open kettle type mixer provided with a heating jacket or other heating means to generate a temperature in the gum base mixture which is the range of 100 to 150°C. The gum base mixing period is generally 2-4 hours per ton of materials.

Evidently, such a prolonged two-step mixing process requires a very high energy consumption and in addition, the separated production of gum base and the subsequent step of compounding the gum base with the remaining chewing gum ingredients involves substantial amounts of operational handling and the use of a range of processing equipment.

In the art, several attempts have therefore been made to develop one-step chewing gum manufacturing processes comprising compounding all the ingredients, including the gum base ingredients, in one single mixing apparatus so as to avoid the separate gum base processing step.

US patent No. 3,440,060 discloses chewing gum products based on the use therein of certain co- and ter-ethylene vinyl acetate gum base polymers. The chewing gum compositions made with these polymers were made in a one-step mixing operation under undefined conditions. However, because of the poor characteristic physical properties of the applied polymers, and the poor gum formulations used in making the chewing gum products disclosed in this patent, such products have little or no current commercial utility because they are too brittle, and are difficult to process.

US patent No. 4,329,369 discloses a one-step process for preparing chewing gum wherein all materials comprising gum base materials including natural resin, vinyl acetate resin, polyisobutylene, ester gum, emulsifier, filler and others on one hand, as well as chewing gum additives including sucrose, glucose, starch hydrolysate, artificial sweetener, flavour, colorant and others on the other hand were charged into a single mixing apparatus and kneaded simultaneously in a single step, however, under elevated pressure in the range of 4 to 10 kg/cm². Using such a process it was possible to carry out the mixing under the pressure at a temperature in the range of 40°C to 60°C and for a period of time in the range of 10 to 15 minutes.

US patent No. 4,968,511 discloses a chewing gum composition comprising, as the sole polymeric component, 5 to 25% by weight of various specific vinyl polyester resin compounds, about 4 to 18% by weight of a plasticizer for the vinyl polyester, about 2 to 11% by weight filler, about 30 to 60% by weight of solid bulk sweetener, about 1 to 25% by weight of liquid bulk sweetener, about 0 to 0.75% by weight of an intense sweetener, about 0.5 to 2.0% by weight of flavouring agent, about 0 to 0.25% by weight of colouring agent and about 0.5 to 5% by weight of emulsifier, and a process for directly preparing a chewing gum product in a one-step mixing process comprising admixing the listed components at a temperature of about 50 to 100°C at atmospheric pressure for 20-45 minutes.

Further methods for continuous production of chewing gum by using a single high efficiency mixer are disclosed in e.g. documents US 6 017 565 and US 5 976 581, which does not require the separate manufacture of chewing gum base and other chewing gum ingredients.

Therefore, it would appear that up till now, no generally applicable process permitting a one-step compounding and mixing of all gum base ingredients and all chewing gum additives has not been available.

Currently applied gum base elastomeric and resinous materials are generally poorly degradable implying that chewing gum made from such materials gives rise to environmental pollution as used chewing gum will persist under indoor and outdoor environmental conditions for prolonged periods of time. Recently, chewing gum formulations having improved properties with regard to degradability has been disclosed, e.g. in US patent No. 5,672,367. where the claimed chewing gum comprises at least one degradable polyester polymer obtained by the polymerisation of cyclic esters, e.g. based on lactides, glycolides, trimethylene carbonate and ε-caprolactone. According to this patent, the chewing gum is made from such degradable polymers in a two-step process including the preparation of the gum base, melting the gum base and admixing the melted gum base with the chewing gum additives.

The degradable polymers disclosed in the above patent preferably have unstable bonds in the polymer chain, which are prone to be broken hydrolytically or under the influence of light. These characteristics, however, render the polymers susceptible to degradation at elevated temperatures such as at their melting temperatures which are typically in the range of about 50°C to about 100°C.

### SUMMARY OF THE INVENTION

It has now been found that it is possible to apply one-step processes carried out at atmospheric pressure for manufacturing chewing gum of any conventional

composition to thereby obtain chewing gum products, including such products containing in the chewing gum base one or more biologically or environmentally degradable polymers, having excellent sensory and other quality parameters, which generally exceed those obtained when making similar chewing gum products in a conventional two-step process including a step of melting the gum base. A major object of the present invention is therefore to provide a generally applicable, cost-effective and gentle process for making chewing gum of a high masticatory quality which is retained for extended periods of time, using a single mixing step performed at atmospheric pressure.

It is an primary object of the present invention to provide a process for preparing chewing gum which process, relative to conventional processes herefore, is more cost effective and requires less processing equipment.

Accordingly, the invention pertains to a process for preparing a chewing gum, the process comprising charging, in an appropriate order, all of the gum base components and all of the chewing gum additives into a mixing apparatus and operating the apparatus at atmospheric pressure to obtain the chewing gum, subject to the limitation that the gum base does not contain a vinyl polyester as the sole functional chewing gum polymer.

In certain particularly useful embodiments, the process is one wherein the gum base comprises at least one environmentally degradable or biodegradable polymer such as a polyester, a polycarbonate, a polyester amide, a polypeptide, an amino acid homopolymer or a protein.

When applying the one-step process according to the invention, a chewing gum having improved consistence, e.g. socalled volume, softness.

One of many features of one embodiment of the invention is that the one-step process relies somewhat more on the mechanical processing of the gum components than the prior art techniques rather than dedicated heating, e.g. premelting of the gumbase components.

According to a further preferred embodiment of the invention, the heating of the gum base components is primarily provided as a result of the mechanical friction during mixing.

According to the invention, an extremely simple process has been obtained. Not only is the process less time- and manpower consuming, but it also facilitates an improved overall process logistic. This is among other things due to the fact that different parallel and/or serial interdependent mixing processes may be avoided partly or completely, thereby facilitating improved stock management, etc.

Moreover, this improved logistic facilitate an improved traceability in the sense that specific ingredients may be traced in each mix, and in principle to each chewing gum with relatively simple measures..

Moreover, due to the simple measure of the invention, the mixing process may be performed by operators having less skills with respect components heating and pressure management. When operating during almost atmospheric pressure and typically relatively low temperature, great safety improvement has been obtained.

Moreover, when dealing with processing of chewing gum based completely or partly biodegradable polymers, the overall obtainable low process temperature may spare the vulnerable biodegradable polymer, thereby obtaining increased control of the polymer properties.

Moreover, variability of product quality may be reduced due the single step operation.

Moreover, manufacturing time will be extremely reduced.

Moreover, the process may be simplified, so that investment cost may be extremely reduced and a significant retionalization may be achieved.

Moreover, the gum base raw materials are never subjected to the high temperature, so that deterioration of quality such as pyrolytic odour and taste may be prevented.

### DETAILED DESCRIPTION OF THE INVENTION

In the one-step process according to the invention, all of the gum base components and all of the chewing gum additives are initially charged, in an appropriate order, into a mixing apparatus and mixed herein while operating the apparatus at atmospheric pressure to obtain the chewing gum. As used herein, the expression "one-step process" includes a process where two or more gum base components are added to the mixing apparatus in a non-melted or non-heated form.

In the context of the present invention, the expression "atmospheric pressure" denotes that the pressure is close to atmospheric pressure. It may be that the pressure is slightly outside the atmospheric pressure. In ranges this may be from 0.90 to 1.10 Pascal, more preferably from 0.95 to 1.05 Pascal. Most preferably the pressure is atmospheric pressure (i.e. 1 Pascal).

In the context of the present invention, the expression "in an appropriate order" implies that all of the components may be added simultaneously or that all or a part of certain components are added first followed by mixing for a selected period of time followed in turn by the addition, under continuous mixing, of all or some of the remaining components or parts thereof until all the components have been loaded into the mixing apparatus.

As used herein, the expressions "gum base component" refers to any component that 0 is conventionally used in the industry to provide the generally water insoluble part of the chewing gum, generally being referred to as the gum base, that determines i.a. the masticatory properties of the final chewing gum product and which typically constitutes 10 to 99% by weight of the total chewing gum formulation. The expression "functional chewing gum polymer" as also used herein refers to a polymeric compound used as a gum base compound as defined herein.

Typically, a chewing gum base formulation comprises one or more elastomeric compounds which may be of synthetic or natural origin, one or more resinous compounds, one or more elastomer plasticizers, fillers, softening compounds and minor amounts of miscellaneous ingredients such as antioxidants and colorants, etc.

Useful synthetic elastomers include, but are not limited to, synthetic elastomers listed in Food and Drug Administration, CFR, Title 21, Section 172,615, the Masticatory Substances, Synthetic) such as polyisobutylene. e.g. having a gas pressure chromatography (GPC) average molecular weight in the range of about 10,000 to about 1,000,000 including the range of 50,000 to 80,000, isobutylene-isoprene copolymer (butyl elastomer), styrene-butadiene copolymers e.g. having styrene-butadiene ratios of about 1:3 to about 3:1, polyvinyl acetate (PVA), e.g. having a GPC average molecular weight in the range of 2,000 to about 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to about 50% by weight such as 10 to 45% by weight of the copolymer, and combinations hereof.

It is common in the industry to combine in a gum base a synthetic elastomer having a high molecular weight and a low-molecular-weight elastomer. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Particularly interesting elastomeric or resinous polymer compounds which advantageously can be used in a process according to the invention include polymers which, in contrast to currently used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

Accordingly, in one preferred embodiment at least one component of the gum base is an environmentally degradable or biodegradable polymer. In the present context, the expression "environmentally degradable or biodegradable polymer" refers to a chewing gum base component which, after dumping the chewing gum, is capable of undergoing a physical, chemical and/or biological degradation whereby the dumped chewing gum waste becomes more readily removable from the site of dumping or is eventually disintegrated to lumps or particles which are no longer recognisable as being chewing gum remnants. The degradation or disintegration of such degradable polymers can be effected or induced by physical factors such as temperature, light, moisture, by chemical factors such as hydrolysis caused by a change in pH or by the action of enzymes capable of degrading the polymers. In other useful embodiments all of the polymer components of the gum base are environmentally degradable or biodegradable polymers.

Suitable examples of environmentally or biologically degradable chewing gum base polymers include degradable polyesters, polycarbonates, polyester amides, polypeptides, homopolymers of amino acids such as polylysine, and proteins including derivatives hereof. Particularly useful compounds of this type include polyester polymers obtained by the polymerisation of one or more cyclic esters as disclosed in US patent No. 5,672,367 which is incorporated herein by reference. These polymers include polymers based on one or more cyclic esters selected from lactides, glycolides, trimethylene carbonates and ε-caprolactone.

In the present context, useful natural elastomers include the elastomers listed in Food and Drug Administration, CFR, Title 21, Section 172,615, as "Masticatory Substances of Natural Vegetable Origin" including natural rubber compounds such as smoked or liquid latex and guayule and other natural gums including jelutong, lechi caspi, massaranduba balata, sorva, perillo, rosindinha, massaranduba chocolate, chicle, nispero, gutta hang kang, and combinations thereof. The preferred synthetic elastomer and natural elastomer concentrations vary depending on whether the chewing gum in which the base is used is adhesive or conventional, bubble gum or regular gum, as discussed below. Presently preferred natural elastomers include jelutong, chicle, massaranduba balata and sorva.

In accordance with the invention, the chewing gum base components which are used herein may include one or more resinous compounds contributing to obtain the desired masticatory properties and acting as plasticizers for the elastomers of the gum base composition. In the present context, useful elastomer plasticizers include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of elastomer plasticizers will vary depending on the specific application, and on the type of elastomer(s) being used.

A chewing gum base formulation may, if desired, include one or more fillers/texturisers including as examples, magnesium and calcium carbonate, sodium sulphate, ground limestone, silicate compounds such as magnesium and aluminium silicate, kaolin and clay, aluminium oxide, silicium oxide, talc, titanium oxide, mono-, di- and tri-calcium phosphates, cellulose polymers, such as wood, and combinations thereof.

The fillers/texhuisers may also include natural organic fibres such as fruit vegetable fibres, grain, rice, cellulose and combinations thereof.

A gum base formulation may, in accordance with the present invention comprise one or more softeners e.g. sucrose polyesters including those disclosed in WO 00/25598, which is incorporated herein by reference, tallow, hydrogenated fat including tallow, hydrogenated and partially hydrogenated vegetable oils, cocoa butter, glycerol monostearate, glycerol triacetate, lecithin, mono-, di- and triglycerides, acetylated monoglycerides, fatty acids (e.g. stearic, palmitic, oleic and linoleic acids), and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Mono- and diglycerides of edible fatty acids, lactic acid esters and acetic acid esters of mono and diglycerides of edible fatty acids, acetylated mono and diglycerides, sugar esters of edible fatty acids, Na-, K-, Mg- and Ca-stearates, lecithin, hydroxylated lecithin and the like are examples of conventionally used emulsifiers which can be added to the chewing gum base. In case of the presence of a biologically or pharmaceutically active ingredient as defined below, the formulation may comprise certain specific emulsifiers and/or solubilisers in order to enhance dispersion and release of the active ingredient

Waxes and fats are conventionally used for the adjustment of the consistency and for softening of the chewing gum base when preparing chewing gum bases. In connection with the present invention, any conventionally used and suitable type of wax and fat may be used, such as for instance rice bran wax, polyethylene wax, petroleum wax (refined paraffin and microcrystalline wax), paraffin, bees' wax, carnauba wax, candelilla wax, cocoa butter, degreased cocoa powder and any suitable oil or fat, as e.g. completely or partially hydrogenated vegetable oils or completely or partially hydrogenated animal fats.

In a further embodiment of the present invention the gum base is wax free.

Furthermore, the gum base formulation may, in accordance with the present invention, comprise colorants and whiteners such as FD&C-type dyes and lakes, fruit and vegetable extracts, titanium dioxide and combinations thereof. Further useful chewing gum base components include antioxidants, e.g. butylated hydroxytoluene (BHT), butyl hydroxyanisol (BHA), propylgallate and tocopherols, and preservatives.

The composition of chewing gum base formulations which in the present one-step process are admixed with chewing gum additives as defined below can vary substantially depending on the particular product to be prepared and on the desired masticatory and other sensory characteristics of the final product. However, typical ranges (% by weight) of the above gum base components are: 5 to 100% elastomeric compounds, 5 to 55% elastomer plasticizer, 0 to 50% filler/texturiser, 5 to 35% softener and 0 to 1% of miscellaneous ingredients such as antioxidants, colorants, etc.

In accordance with the invention, the one-step process involves that gum base components as described above are loaded into a mixing apparatus with chewing gum additives. In the present context, the term "chewing gum additive" is used to designate any component, which in a conventional two-step process is added to the separately produced and pre-melted or -heated gum base. The major proportion of such conventionally used additives are water soluble, but water-insoluble components, such as e.g. water-insoluble flavouring compounds, can also be included.

In the present context, chewing gum additives include bulk sweeteners, high intensity sweeteners, flavouring agents, softeners, emulsifiers, colouring agents, binding agents, acidulants, fillers, antioxidants and other components such as pharmaceutically or biologically active substances, that confer desired properties to the finished chewing gum product.

Suitable bulk sweeteners include both sugar and non-sugar components. Bulk sweeteners typically constitute from about 5 to about 95% by weight of the chewing gum, more typically about 20 to about 80% by weight such as 30 to 60% by weight of the gum.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High intensity artificial sweetening agents can also be used alone or in combination with the above sweeteners. Preferred high intensity sweeteners include, but are not limited to sucralose, aspartame, salts of acesulfame, alitame, saccharin and its salts, cyclamic acid and its salts, glycyrrhizin, dihydrochalcones, thaumatin, monellin, sterioside and the like, alone or in combination. In order to provide longer lasting sweetness and flavour perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fibre extrusion may be used to achieve desired release characteristics. Encapsulation of sweetening agents can also be provided e.g. using as the encapsulation agent another chewing gum component such as a resinous compound.

Usage level of the artificial sweetener will vary considerably depending e.g. on factors such as potency of the sweetener, rate of release, desired sweetness of the product, level and type of flavour used and cost considerations. Thus, the active level of artificial sweetener may vary from about 0.02 to about 8% by weight. When carriers used for encapsulation are included, the usage level of the encapsulated sweetener will be proportionately higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie gum is desired, a low caloric bulking agent can be used. Examples of low caloric bulking agents include polydextrose, Raftilose, Raftilin, fructooligosaccharides (NutraFlora^{®}, palatinose oligosaccharided; guar gum hydrolysates (e.g. Sun Fiber^{®}) or indigestible dextrins (e.g. Fibersol^{®}). However, other low calorie-bulking agents can be used.

Further chewing gum additives which may be included in the chewing gum mixture processed in the present process include surfactants and/or solubilisers, especially when pharmaceutically, cosmetically or biologically active ingredients are present. As examples of types of surfactants to be used as solubilisers in a chewing gum composition according to the invention reference is made to H.P. Fiedler, Lexikon der Hilfstoffe für Pharmacie, Kosmetik und Angrenzende Gebiete, page 63-64 (1981) and the lists of approved food emulsifiers of the individual countries. Anionic, cationic, amphoteric or non-ionic solubilisers can be used. Suitable solubilisers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyl-latylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. The expression "solubiliser" is used in the present text to describe both possibilities, the solubiliser used must be suitable for use in food and/or medicine.

In the presence of an active ingredient the chewing gum may preferably also comprise a carrier known in the art.

One significant advantage of the present one-step mixing process is that the temperature throughout the entire operation can be kept at a relatively low level such as it will be described in the following. This is an advantageous feature with regard to preserving the aroma of added flavouring components which may be prone to deterioration at higher temperatures. Aroma agents and flavouring agents which are useful in a chewing gum produced by the present process are e.g. natural and synthetic flavourings (including natural flavourings) in the form of freeze-dried natural vegetable components, essential oils, essences, extracts, powders, including acids and other substances capable of affecting the taste profile. Examples of liquid and powdered flavourings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In one preferred embodiment, the flavour is one or more natural flavouring agent(s) which is/are freeze-dried, preferably in the form of a powder, slices or pieces of combinations thereof. The particle size of such agent may be less than 3 mm, such as less than 2 mm, more preferred less than 1 mm, calculated as the longest dimension of the particle. The natural flavouring agent may also be in a form where the particle size is from about 3 µm to 2 mm, such as from 4 µm to 1 mm. Preferred natural flavouring agents include seeds from a fruit e.g. from strawberry, blackberry and raspberry.

Various synthetic flavours, such as mixed fruit flavour may also be used according to the present invention. As indicated above, the aroma agent may be used in quantities smaller than those conventionally used. The aroma agents and/or flavours may be used in an amount of from 0.01 to about 30% by weight of the final product depending on the desired intensity of the aroma and/or flavour used. Preferably, the content of aroma/flavour is in the range of from 0.2 to 3% by weight of the total composition.

In one embodiment the chewing gum composition processed in a one-step operation according to the invention comprises a pharmaceutically, cosmetically or biologically active substance. Examples of such active substances, a comprehensive list of which is found e.g. in WO 00/25598, which is incorporated herein by reference, include drugs, dietary supplements, antiseptic agents, pH adjusting agents, anti-smoking agents (e.g. nicotine) and substances for the care or treatment of the oral cavity and the teeth such as hydrogen peroxide and compounds capable of releasing urea during chewing. Examples of useful active substances in the form of antiseptics include salts and derivatives of guanidine and biguanidine (for instance chlorhexidine diacetate) and the following types of substances with limited water-solubility: quaternary ammonium compounds (e.g. ceramine, chloroxylenol, crystal violet, chloramine), aldehydes (e.g. paraformaldehyde), derivatives of dequaline, polynoxyline, phenols (e.g. thymol, *p*-chlorophenol, cresol), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, The Extra Pharmacopoeia, 28th edition, page 547-578; metal salts, complexes and compounds with limited water-solubility, such as aluminium salts, (for instance aluminium potassium sulphate AlK(SO₄)₂,12H₂O) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodium monofluorophosphate, aminofluorides, stannous fluoride), phosphates, carbonates and selenium. Further active substances can be found in J. Dent.Res. Vol. 28 No. 2, page 160-171,1949.

Examples of active substances in the form of agents adjusting the pH in the oral cavity include: acids, such as adipinic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulphates or oxides of sodium, potassium, ammonium, magnesium or calcium, especially magnesium and calcium.

In accordance with the invention, the present one-step process comprises, subsequent to or during the step of charging, in any appropriate order, all of the gum base components and all of the chewing gum additives into a mixing apparatus, a step of operating the apparatus under atmospheric pressure conditions to obtain the chewing gum. In general terms, a typical one-step batch mixing operation is carried out as described in the following.

All of the chewing gum components selected for the particular type of chewing gum to be processed are mixed thoroughly in any conventional type of kneading or mixing vessel such as e.g. a kettle provided with mixing means such as e.g. horizontally placed Z-shaped arms, which are capable of intimately mixing the selected chewing gum components to produce a homogeneous chewing gum mass. It is also possibly to carry out a batchwise one-step processing of the chewing gum using an shear stress generating apparatus including an extruder apparatus. Preferably, the mixing vessel is provided with heating means such as a heating jacket permitting hot water, oil or steam to be circulated around the mixing space, or heating elements. Generally, the temperature is set initially at a temperature in the range of 30-80°C, the preferred temperature depending e.g. on the temperature at which the selected elastomeric or resinous polymers become sufficiently soft to be processed. A typical initial mixing temperature is in the range of 40 to 65°C such as the range of 45 to 60°C. It is preferred that the temperature does not rise substantially during the mixing operation, e.g. not more than 5-20°C. In preferred embodiments, the temperature does not exceed about 60°C at any point in time during the batch mixing process.

Typically, the mixing process starts with mixing of the gum base components in the quantities that have been weighed out, and the processing of these components continues for 1-30 minutes, such as 5-20 including about 10 minutes following which the sweetening component(s) in powder form or in liquid form is/are added. Typically, the time period for dosing of sweeteners and the subsequent processing is in the range of 1 to 20 minutes such as in the range 2 to 15 minutes, e.g. about 7 minutes. When polymers of a high molecular weight is used, it may be required to extend the mixing period to e.g. up to 40 minutes, optionally while cooling the mixing space of the mixing apparatus.
However, it should be noted that the mixing step may be extended to a certain degree in time when applying conventional non-degradable gum base raw materials.

The flavours and the remaining chewing gum components are typically added to the mixing apparatus still being continuously operated following the addition of sweetener and mixing hereof into the gum base component, and mixing is continued for a further 1 to 10 minutes, such as 2 to 8 minutes, typically about 5 minutes. The admixture of flavours and the remaining components may also take place in the beginning of the kneading process, i.e. before the admixture of the sweeteners. It is also possible to add the selected amount of flavours in two or more rounds throughout the kneading/mixing process. It is preferred that the total mixing step is completed within 30 minutes such as within 20 minutes or even within 15 minutes of operation.

However, it should be noted that the mixing step may be extended to a certain degree in time when applying conventional non-degradable gum base raw materials.

In a further embodiment, the invention for manufacturing conventional chewing gum comprising the step of adding softening system together with low molecular weight polymers. The processing of these components continues for 1-30 minutes, such as 5-20 including about 8 minutes following which high molecular weight polymers and fillers are added. Typically, the time period for dosing and subsequent processing is in the range of 1-10 minutes, such as 2 to 8, typically about 5 minutes. All resins and sweetening powder form are added, and the processing continues for 10-60 minutes, such as 20-40 including about 30 minutes.

The flavours and the remaining chewing gum components are typically added to the mixing apparatus still being continuously operated following the addition of sweetener and mixing hereof into the gum base component, and mixing is continued for a further 1 to 30 minutes, such as 10 to 20 minutes, typically about 15 minutes.

When the kneading/mixing step is completed, the resulting chewing gum mass is recovered from the mixing vessel and e.g. transferred to a cart, tray or the like as it is conventional in industrial chewing gum manufacturing and the gum mass is processed further into finished chewing gum products using conventional steps herefor which are generally known in the art including forming of the chewing gum into cores, sticks, balls, cubes, cylinders and any other desired shape, optionally followed by coating and polishing processes prior to packaging.

Whereas the above description of the process according to the invention refers particularly to a batch process, it is also within the scope of the invention to carry out the one-step mixing process as a continuous process where shear stress is applied to the gum base and additive mixture e.g. using a screw type mixer which can be of the single oscillating or double co-rotating auger types including an extruder of the Buss type. As it is the case with a batch one-step process, the temperature is controlled in a continuous mixing operation so as to secure sufficient softening of the gum base polymers, but without reaching a temperature that may damage the polymers or any other component that is heat sensitive. Thus, the temperature in the mixing chamber of the screw type mixer is preferably within the range of 40 to 80°C such as in the range of 50 to 70°C throughout the entire mixing operation. In preferred embodiments, the temperature does not exceed about 60°C during the continuous mixing operation.

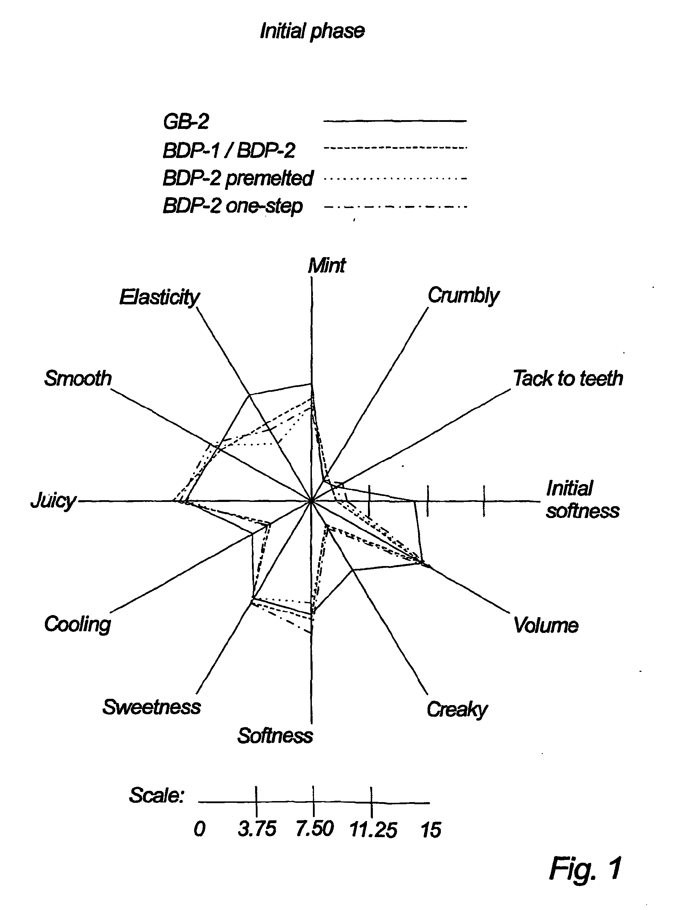

The invention will now be described in the following, non-limiting examples and the drawings wherein
Fig. 1 is a representation of initial phase sensory profile analyses of chewing gum prepared using the one-step process in comparison with chewing gum prepared by a conventional two-step process. GB-2 designates a chewing gum made in a two-step process using the gum base designated herein as gum base B, BDP-1/BDP-2 is a chewing prepared using a one-step process with an equal mixture of degradable polymers designated herein as BDP1 and BDP2, respectively, BDP-2 premelted designates a chewing gum prepared in a two-step process using as the gum base pre-melted degradable polymer BDP2, and BDP-2 one-step designates a chewing gum prepared in a one-step process according to the invention, using non-melted degradable polymer BDP2,
Fig. 2 is a representation of intermediate phase sensory profile analyses of the chewing gums referred to in Fig. 1,
Fig. 3 is a representation of end phase sensory profile analyses of the chewing gums referred to in Fig. 1,
Fig. 4 is a representation showing initial phase sensory analyses of the chewing gums referred to in Fig. 1 as BDP-2 premelted and BDP-2 one-step, respectively,
Fig. 5 is a representation showing intermediate phase sensory analyses of the chewing gums referred to in Fig. 1 as BDP-2 premelted and BDP-2 one-step, respectively,
Fig. 6 is a representation showing end phase sensory analyses of the chewing gums referred to in Fig. 1 as BDP-2 premelted and BDP2 one-step, respectively,
Fig. 7 shows the hardness [N] as measured using an Instron instrument of (i) the chewing gum of Example 7 herein (BDP 2 (one-step)) and (ii) the chewing gum of Example 3 herein (BDP 2 (premelted)), and
Fig. 8 summarises data describing the rheological properties (storage modulus G', determined by a rheometer, type AR1000 from AT Instruments of the chewing gums referred to in Fig. 1 as BDP-2 premelted and BDP-2 one-step, respectively, and a chewing gum comprising non-degradable gum base polymers and made by a conventional two-step process wherein the gum base is prepared prior to admixing with chewing gum additives.
Fig. 9 and 10 summarises data describing the rheological properties (linear viscoelastic region (LVR)) showing storage modulus G', determined by a rheometer, type AR1000 from AT Instruments of different conventional chewing gums made by an one-step process and for comparison made by a conventional two-step process, wherein the different gum bases are prepared prior to admixing with chewing gum additives.
Fig. 11 is a representation showing initial phase sensory analyses of a conventional chewing gum referred to as sample (standard two-step process) and one-step, respectively,
Fig. 12 is a representation showing intermediate phase sensory analyses of a chewing gum referred to as sample (standard two-step process) and one-step, respectively,
Fig. 13 is a representation showing end phase sensory analyses of a chewing gum referred to as sample (standard two-step process) and one-step, respectively,
Fig. 14 is a representation showing in vivo release of nicotine. The graph includes nicotine chewing gums prepared as a conventional two-step process and one-step, respectively.

### EXAMPLE 1

### Preparation of chewing gum with peppermint taste containing degradable gum base polymers using a conventional two-step process wherein the gum base is melted prior to mixing (reference test)

In this example, a degradable polymer as defined in US 5,672,367 and obtained by polymerisation of cyclic esters and having unstable bonds that can be broken hydrolytically or under the influence of light, was used as the gum base part. In the following, the polymer is designated as BDP1. Prior to mixing with the chewing additives as listed below, the gum base polymer was softened/melted in a 100 °C water bath for 30 minutes.

The pre-melted gum base was charged together with about one third of the amount of sorbitol into a conventional double sigma blade mixer (Krupp, Werner & Pfleiderer GmbH, Germany) provided with two blades inside the kettle bowl, each in the shape of the letter "Z", the velocity of which can be set at a velocity in the range of 1 to 110 rpm. In this experiment, the double blade mixer was set at a rotation of 50 rpm. Subsequently, the remaining chewing gum additives as listed in the below Table 1 was added under mixing conditions at the indicated points in time. The composition of the chewing gum formulation and the mixing conditions are summarised in the below Table 1:

**Table 1. Composition of chewing gum with pre-melted degradable gum base polymer BDP1, and mixing conditions**

| Ingredient | Parts (weight) | Time (min.) | Temp (°C) |
|---|---|---|---|
| BDP1 | 40.46 | 0 | 60 |
| Sorbitol powder | 13.26 | 0 | 60 |
| Lecithin | 0.20 | 2 | 58 |
| Lycasine | 5.77 | 2 | 58 |
| Sorbitol powder | 13.46 | 2 | 58 |
| Sorbitol powder | 13.46 | 4 | 55 |
| Peppermint | 1.54 | 6 | 56 |
| Menthol (crystal) | 0.31 | 6 | 56 |
| Menthol powder | 0.37 | 7 | 58 |
| Peppermint powder | 0.19 | 7 | 58 |
| Menthol powder | 0.19 | 7 | 58 |
| Aspartame | 0.19 | 8 | 58 |
| Acesulfame | 0.1 | 8 | 58 |
| Xylitol | 10.8 | 10 | 58 |
| | | | |
| Total | 100.0 | 12 | 58 |

### EXAMPLE 2

### Preparation of chewing gum with peppermint taste containing degradable gum base polymers using a conventional two-step process wherein the gum base is melted prior to mixing (reference test)

In this example, a chewing gum was prepared essentially as described in Example 1, however, with the modification that a different pre-melted degradable polymer, designated BDP2. The composition and the mixing conditions were as listed in the below Table 2:

**Table 2. Composition of chewing gum with pre-melted degradable gum base polymer BDP2, and mixing conditions**

| Ingredient | Parts (weight) | Time (min.) | Temp (°C) |
|---|---|---|---|
| BDP2 | 40.46 | 0 | 63 |
| Sorbitol powder | 13.26 | 0 | 63 |
| Lecithin | 0.20 | 2 | 63 |
| Lycasine | 5.77 | 2 | 63 |
| Sorbitol powder | 13.46 | 2 | 63 |
| Sorbitol powder | 13.46 | 4 | 63 |
| Peppermint | 1.54 | 6 | 63 |
| Menthol (crystal) | 0.31 | 6 | 63 |
| Menthol powder | 0.37 | 7 | 61 |
| Peppermint powder | 0.19 | 7 | 61 |
| Menthol powder | 0.19 | 7 | 61 |
| Aspartame | 0.19 | 8 | 59 |
| Acesulfame | 0.1 | 8 | 59 |
| Xylitol | 10.8 | 10 | 61 |
| | | | |
| Total | 100.0 | 12 | 58 |

### EXAMPLE 3

### Preparation of chewing gum with peppermint taste containing non-degradable polymers using a one-step mixing process according to the invention

In this example, the step of pre-melting the gum base was omitted implying that a conventional gum base designated Gum base A was added directly, i.e. without pre-melting, to the mixing apparatus used in Examples 1-2 and mixed with the chewing gum additives as listed in the below Table 3 at the indicated points in time.

**Table 3. Composition of chewing gum with non-degradable gum base A, and one-step mixing conditions**

| Ingredient | Parts (weight) | Time (min.) | Temp¹ (°C) |
|---|---|---|---|
| Gum base A | 40.46 | 0 | 25 |
| Sorbitol powder | 13.26 | 0 | 25 |
| Lecithin | 0.20 | 2 | 38 |
| Lycasine | 5.77 | 2 | 38 |
| Sorbitol powder | 13.46 | 4 | 42 |
| Sorbitol powder | 13.46 | 5 | 42 |
| Peppermint | 1.54 | 6 | 43 |
| Menthol (crystal) | 0.31 | 6 | 43 |
| Menthol powder | 0.37 | 7 | 45 |
| Peppermint powder | 0.19 | 7 | 45 |
| Menthol powder | 0.19 | 7 | 45 |
| Aspartame | 0.19 | 8 | 45 |
| Acesulfame | 0.1 | 8 | 45 |
| Xylitol | 10.8 | 10 | 46 |
| | | | |
| Total | 100.0 | 12 | 48 |

| | | | |
|---|---|---|---|
| ¹Temperature measured manually in the chewing gum mass before adding new ingredients | | | |

As it appears, it was possible to obtain the final chewing gum using a one-step mixing process within a time period of 12 minutes.

### EXAMPLE 4

### Preparation of chewing gum with peppermint taste containing as the gum base part non-degradable polymers using a one-step mixing process according to the invention

The one-step process of Example 3 was used to prepare another chewing gum comprising as the gum base part, the components of the gum base designated gum base B. The composition and the mixing conditions were as listed in the below Table 4:

**Table 4. Composition of chewing gum with non-degradable gum base B, and one-step mixing conditions**

| Ingredient | Parts (weight) | Time (min.) | Temp (°C) |
|---|---|---|---|
| Gum base B | 40.46 | 0 | 25 |
| Sorbitol powder | 13.26 | 0 | 25 |
| Lecithin | 0.20 | 2 | 38 |
| Lycasine | 5.77 | 2 | 38 |
| Sorbitol powder | 13.46 | 4 | 42 |
| Sorbitol powder | 13.46 | 5 | 43 |
| Peppermint | 1.54 | 6 | 44 |
| Menthol (crystal) | 0.31 | 6 | 44 |
| Menthol powder | 0.37 | 7 | 46 |
| Peppermint powder | 0.19 | 7 | 46 |
| Menthol powder | 0.19 | 7 | 46 |
| Aspartame | 0.19 | 8 | 46 |
| Acesulfame | 0.1 | 8 | 46 |
| Xylitol | 10.8 | 10 | 47 |
| | | | |
| Total | 100.0 | 12 | 48 |

Also with this non-degradable gum base admixed in a non-melted state it was possible to obtain the final chewing gum using a one-step mixing process within a mixing period of 12 minutes.

### EXAMPLE 5

### Preparation of chewing gum with peppermint taste containing as the gum base part degradable polymers using a one-step mixing process according to the invention

The one-step process used in this test was essentially as in Example 4 with the modification, however, that the gum base part was the environmentally degradable polyester polymer designated BDP1 as used in Example 1. The composition and the mixing conditions were as listed in the below Table 5:

**Table 5. Composition of chewing gum with degradable gum base polymer BDP1. and one-step mixing conditions**

| Ingredient | Parts (weight) | Time (min.) | Temp (°C) |
|---|---|---|---|
| Gum base polymer BDP1 | 40.46 | 0 | 25 |
| Sorbitol powder | 13.26 | 0 | 25 |
| Lecithin | 0.20 | 2 | 43 |
| Lycasine | 5.77 | 2 | 43 |
| Sorbitol powder | 13.46 | 2 | 43 |
| Sorbitol powder | 13.46 | 3 | 48 |
| Peppermint | 1.54 | 4 | 51 |
| Menthol (crystal) | 0.31 | 4 | 51 |
| Menthol powder | 0.37 | 5 | 51 |
| Peppermint powder | 0.19 | 5 | 51 |
| Menthol powder | 0.19 | 5 | 51 |
| Aspartame | 0.19 | 6 | 51 |
| Acesulfame | 0.1 | 6 | 51 |
| Xylitol | 10.8 | 8 | 53 |
| | | | |
| Total | 100.0 | 10 | 53 |

As it appears, it was possible to mix all of the chewing gum components in a one-step mixing process within the same time period as in the corresponding reference two-step process of Example 1 and without reaching a higher temperature in the final chewing gum mass. Indeed, the final temperature in the final chewing gum mass was significantly lower when using a one-step process.

### EXAMPLE 6

### Preparation of chewing gum with peppermint taste containing as the gum base part degradable polymers using a one-step mixing process according to the invention

The one-step process used in this Example was essentially as that of Example 5 with the modification, however, that the gum base part was a different environmentally degradable polyester polymer designated BDP2 and having the same basic characteristics as the polymer used in Example 5. The composition and the mixing conditions were as listed in the below Table 6:

**Table 6. Composition of chewing gum with degradable gum base polymer BDP2, and one-step mixing conditions**

| Ingredient | Parts (weight) | Time (min.) | Temp (°C) |
|---|---|---|---|
| Gum base polymer BDP2 | 40.46 | 0 | 25 |
| Sorbitol powder | 13.26 | 0 | 25 |
| Lecithin | 0.20 | 2 | 38 |
| Lycasine | 5.77 | 2 | 38 |
| Sorbitol powder | 13.46 | 2 | 43 |
| Sorbitol powder | 13.46 | 3 | 50 |
| Peppermint | 1.54 | 4 | 50 |
| Menthol (crystal) | 0.31 | 4 | 50 |
| Menthol powder | 0.37 | 5 | 50 |
| Peppermint powder | 0.19 | 5 | 50 |
| Menthol powder | 0.19 | 5 | 50 |
| Aspartame | 0.19 | 6 | 50 |
| Acesulfame | 0.1 | 6 | 50 |
| Xylitol | 10.8 | 8 | 54 |
| | | | |
| Total | 100.0 | 10 | 54 |

The mixing time required and the final chewing gum mass temperature were essentially as those obtained for degradable polymer BDP1when used in a one-step process.

### EXAMPLE 7

### Preparation of chewing gum with peppermint taste containing as the gum base part a mixture of degradable polymers using a one-step mixing process according to the invention

The chewing gum in this example was prepared essentially as described in Examples 5 or 6, however with the modification that equal amounts of degradable polymer BDP1 and degradable polymer BDP2, respectively was used as the gum base part instead of either of the individual degradable polymers. The composition and the mixing conditions were as listed in the below Table 7:

**Table 7. Composition of chewing gum with equal amounts of degradable gum base polymers BDP1 and BDP2, and one-step mixing conditions**

| Ingredient | Parts (weight) | Time (min.) | Temp (°C) |
|---|---|---|---|
| Gum base polymer BDP1 | 20.23 | 0 | 25 |
| Gum base polymer BDP2 | 20.23 | 0 | 25 |
| Sorbitol powder | 13.26 | 0 | 25 |
| Lecithin | 0.20 | 2 | 35 |
| Lycasine | 5.77 | 2 | 35 |
| Sorbitol powder | 13.46 | 2 | 35 |
| Sorbitol powder | 13.46 | 3 | 48 |
| Peppermint | 1.54 | 4 | 50 |
| Menthol (crystal) | 0.31 | 4 | 50 |
| Menthol powder | 0.37 | 5 | 53 |
| Peppermint powder | 0.19 | 5 | 53 |
| Menthol powder | 0.19 | 5 | 53 |
| Aspartame | 0.19 | 6 | 53 |
| Acesulfame | 0.1 | 6 | 53 |
| Xylitol | 10.8 | 8 | 53 |
| | | | |
| Total | 100.0 | 10 | 52 |

The mixing time required and the final chewing gum mass temperature were essentially as those obtained when using either of degradable polymers BDP1 and BDP2 separately.

### EXAMPLE 8

### Preparation of chewing gum with peppermint taste using as the gum base a mixture of non-degradable and degradable polymers applying a one-step mixing process wherein all of the gum base components are loaded separately to the mixing apparatus

In this Example, the gum base part consisted of both a mixture of non-degradable polymers, i.e. polyisobutylene, polyvinyl acetate having low molecular weight and an ester gum, and degradable polymer BDP1. Each of these chewing gum base components was added separately to the mixing apparatus used in the previous Examples at the points in time indicated in table 8 below:

**Table 8. Composition of chewing gum with a bum base mixture of non-degradable gum base polymers added separately and the degradable gum base polymer BDP1, and one-step mixing conditions**

| Ingredient | Parts (weight) | Time (min.) | Temp (°C) |
|---|---|---|---|
| Gum base polymer BDP1 | 12.40 | 0 | 35 |
| Polyisobutylene | 4.10 | 0 | 35 |
| Talc | 5.00 | 0 | 35 |
| Polyvinyl acetate, LW | 6.20 | 0 | 35 |
| Ester gum | 4.35 | 1 | 35 |
| Monodiglycerides | 3.75 | 2 | 48 |
| Hydrogenated fat | 5.10 | 2 | 48 |
| Sorbitol powder | 13.26 | 2 | 48 |
| Lecithin | 0.20 | 3 | 52 |
| Lycasine | 5.77 | 3 | 52 |
| Sorbitol powder | 13.42 | 3 | 52 |
| Sorbitol powder | 13.42 | 4 | 53 |
| Peppermint | 1.54 | 5 | 54 |
| Menthol (crystal) | 0.31 | 5 | 54 |
| Menthol powder | 0.37 | 6 | 54 |
| Peppermint powder | 0.19 | 6 | 54 |
| Menthol powder | 0.19 | 6 | 54 |
| Aspartame | 0.19 | 7 | 54 |
| Acesulfame | 0.10 | 7 | 54 |
| Xylitol | 10.78 | 8 | 54 |
| | | | |
| Total | 100.0 | 10 | 54 |

As it appears, it is possible to provide a one-step chewing gum mixing process wherein a range of non-degradable gum base polymers and degradable polymes are added separately in the process and obtain the finished chewing mass within a very short period of time.

### EXAMPLE 9

### Preparation of chewing gum with peppermint taste containing as the gum base part degradable polymers using a one-step mixing process according to the invention

The one-step process used in this test was essentially as in Example 5 with the modification, however, a smaller amount of the degradable gum base polymer was used and the omitted amount replaced by a filler and hydrogenated fat. The composition and the mixing conditions were as listed in the below Table 9 summarising the composition and the mixing conditions:

**Table 9. Composition of chewing gum with degradable gum base polymer BDP1, and one-step mixing conditions**

| Ingredient | Parts (weight) | Time (min.) | Temp (°C) |
|---|---|---|---|
| BDP 1 | 30.46 | 0 | 28 |
| Sorbitol powder | 13.26 | 0 | 28 |
| Filler | 5.0 | 1 | 38 |
| Hydrogenated fat | 5.0 | 1 | 38 |
| Sorbitol powder | 13.46 | 1 | 38 |
| Lecithin | 0.20 | 3 | 41 |
| Lycasine | 5.77 | 3 | 41 |
| Sorbitol powder | 13.46 | 3 | 41 |
| Peppermint | 1.54 | 5 | 43 |
| Menthol (crystal) | 0.31 | 5 | 43 |
| Menthol powder | 0.37 | 6 | 46 |
| Peppermint powder | 0.19 | 6 | 46 |
| Menthol powder | 0.19 | 6 | 46 |
| Aspartame | 0.19 | 7 | 46 |
| Acesulfame | 0.1 | 7 | 46 |
| Xylitol | 10.8 | | 46 |
| | | | |
| Total | 100.0 | 10 | 46 |

### EXAMPLE 10

### Sensory profile analyses of test chewing gum

The following chewing gums were tested by a sensory panel of 5 panellists extensively trained in sensory testings of chewing gum: (i) the chewing gum of Example 4, i.e. a chewing gum made in a one-step process using the gum base designated herein as gum base B, (ii) the chewing gum of Example 7, i.e. a chewing prepared using a one-step process with an equal mixture of degradable polymers BDP1 and BDP2, (iii) the chewing gum of Example 2, i.e. a chewing gum prepared in a two-step process using as the gum base pre-melted degradable polymer BDP2 and (iv) the chewing gum of Example 6, i.e. a chewing gum prepared in a one-step process according to the invention, using non-melted degradable polymer BDP2.

The samples of chewing gum was tested by serving them to the sensory panellists in tasting booths made in accordance with ISO 8598 standards at room temperature in 40 ml tasteless plastic cups with randomised 3-figure codes. Test samples were evaluated after chewing for 0-1 minutes (initial phase), 2-3 minutes (intermediate phase) and 4-5 minutes (end phase), respectively. Between each sample tested, the panellists were allowed a brake of 3 minutes.

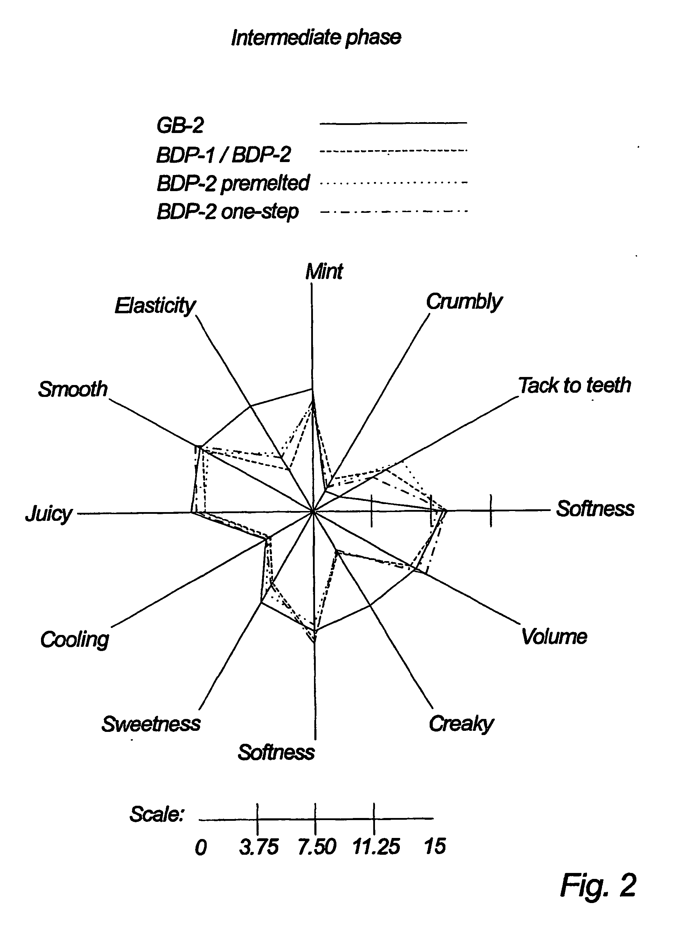

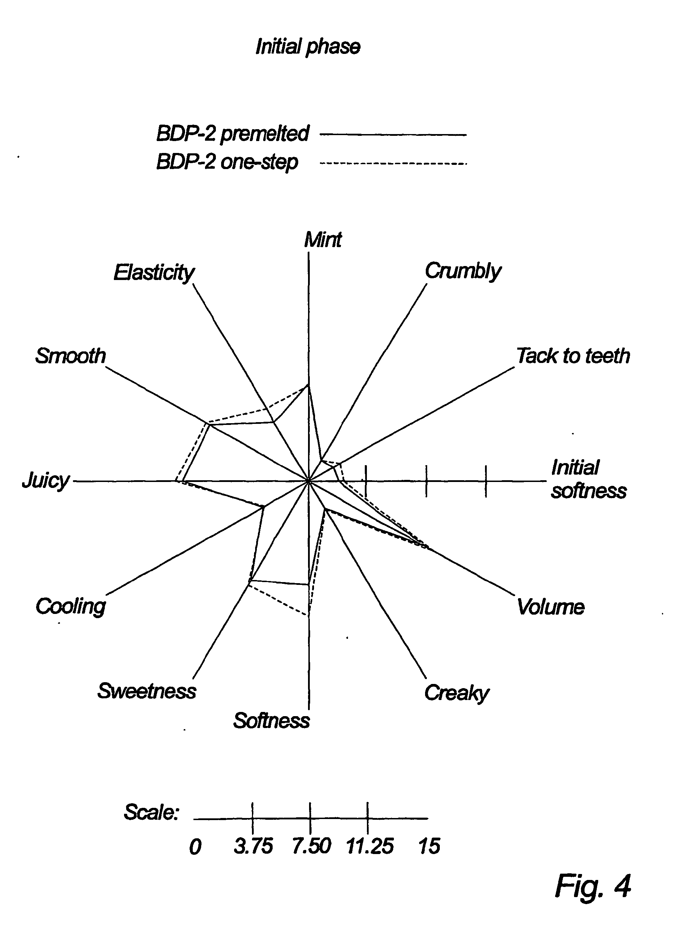

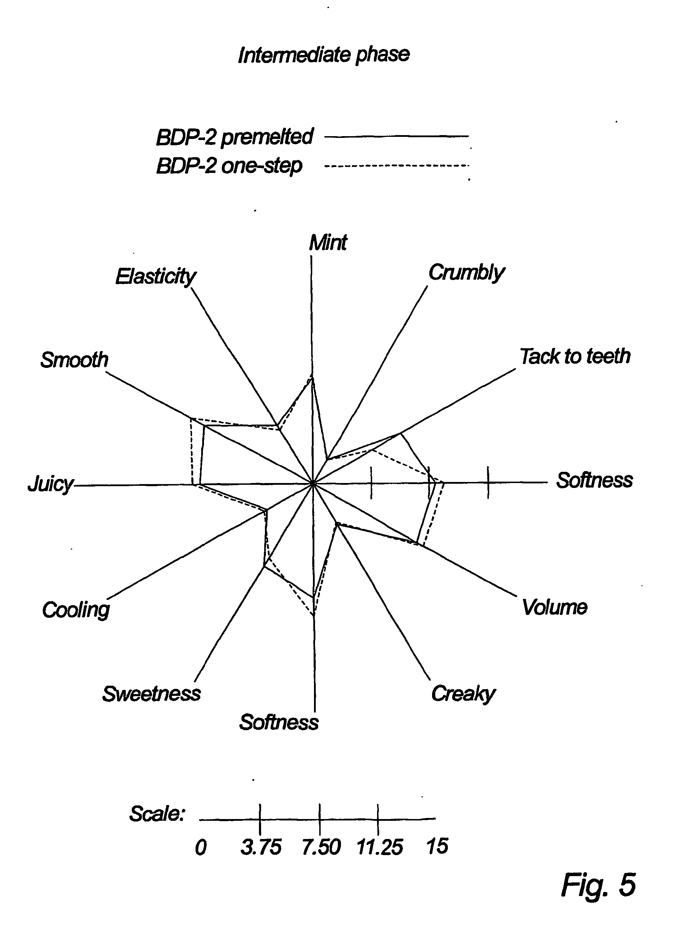

The following standard parameters were assessed: Mint flavour, crumble ness, tacking to teeth, initial softness, volume, creakiness, softness, sweetness, cooling effect, juiciness, smoothness and elasticity. For each of these parameters, the panellists were required to provide their assessments according to an arbitrary scale of 0-15. The data obtained were processed using a FIZZ computer program (French Bio System) and the results were transformed to sensory profile diagrams as shown in Figures 1-6. Additionally, the sensory profile test data were subjected to statistical analyses, the results of which are summarised in Tables 10-12 below.

The major differences between test chewing gums in the initial testing phase were the following:

Chewing gums prepared by a one-step process according to the invention showed a significantly higher softness than corresponding chewing gums prepared in a conventional two-step process, i.e. including a pre-melting step of the gum base.

Chewing gum prepared using a one-step according to the invention had a significantly larger volume than corresponding chewing gum prepared in a conventional two-step process.

The major differences between test chewing gums in the intermediate testing phase were the following:

Chewing gums prepared using the one-step process of the invention showed significantly less tacking to teeth, were significantly softer and had a significantly larger volume than gums prepared in a conventional two-step process.

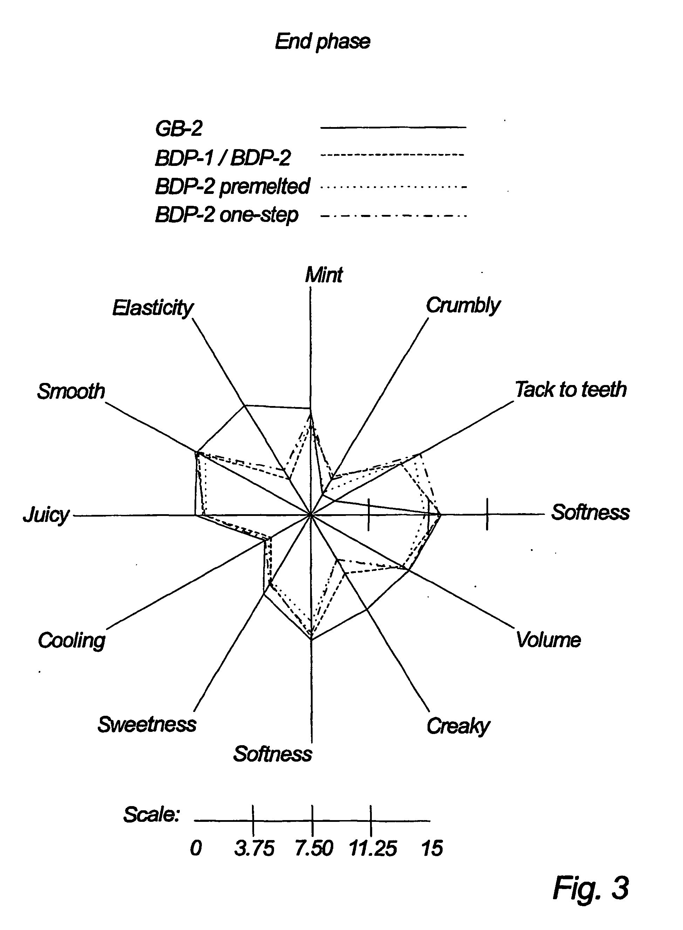

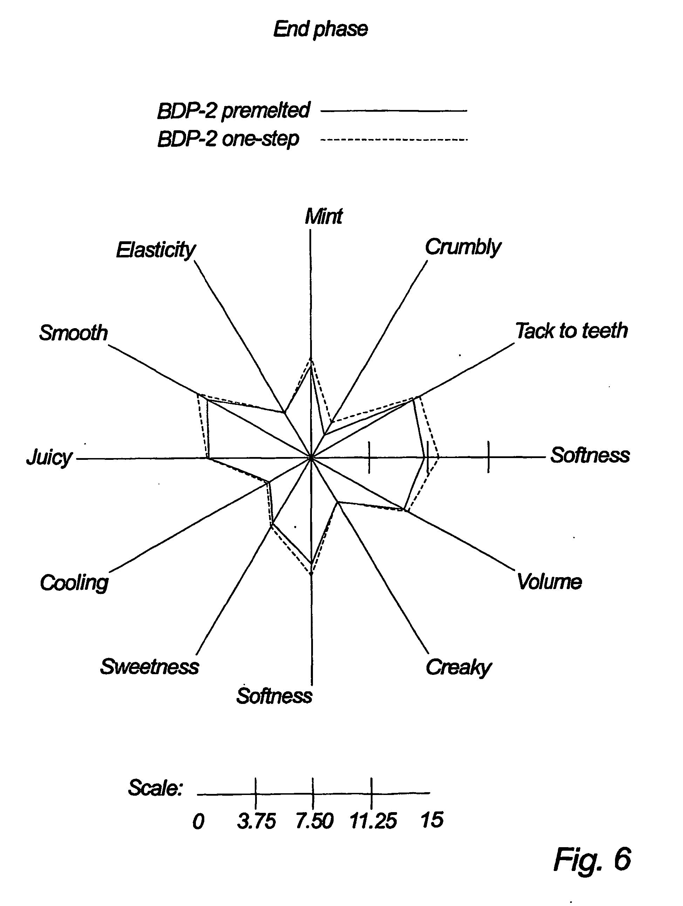

Testing in the end phase did not reveal statistically significant differences between samples, but as it appears from figures 3 and 6, the samples prepared using the present one-step process had a higher softness and a larger volume.

It can therefore be concluded that the use of a one-step chewing gum process without a separate gum base preparation and/or a pre-melting step prior to admixture with the chewing gum additives results in final chewing gum products that, relative to chewing gum processed in two separate mixing steps, are superior with regard to essential sensory characteristics.

**Table 10. Statistical analyses of sensory profile analyses of test chewing gums, initial phase**

| | Initial Phase | | | | |
|---|---|---|---|---|---|
| | Gum base B | BDP1/BDP2 | BDP2, premelted | BDP2, one-step | Significance level¹ |
| Initial softness | A | B | B | B | *** |
| Tacking to teeth | | | | | NS² |
| Crumbleness | B | A | B | B | ***/*** |
| Mint flavour | A | AB | B | B | */* |
| Elasticity | A | B | B | B | ** |
| Smoothness | AB | B | AB | A | */** |
| Juiciness | | | | | NS |
| Cooling | A | B | B | B | **/** |
| Sweetness | | | | | NS |
| Softness | AB | AB | B | A | ***** |
| Creakiness | A | B | B | B | ***/** |
| Volume | B | B | B | A | ** |

**Table 11. Statistical analyses of sensory profile analyses of test chewing gums, intermediate phase**

| | Intermediate Phase | | | | |
|---|---|---|---|---|---|
| | Gum base B | BDP1/BDP2 | BDP2, premelted | BDP2, one-step | Significance level |
| Softness | | | | | NS |
| Tacking to teeth | C | B | A | B | ***/*** |
| Crumbleness | | | | | NS |
| Mint flavour | | | | | NS (0,0715) |
| Elasticity | A | C | B | BC | *** |
| Smoothness | AB | BC | C | A | **/*** |
| Juiciness | | | | | NS |
| Cooling | | | | | NS |
| Sweetness | A | B | AB | B | * |
| Softness | | | | | NS |
| Creakiness | A | B | B | B | *** |
| Volume | BC | C | B | A | ** |

**Table 12. Statistical analyses of sensory profile analyses of test chewing gums, end phase**

| | End Phase | | | | |
|---|---|---|---|---|---|
| | Gum base B | BDP1/BDP2 | BDP2, premelted | BDP2, one-step | Significance level |
| Softness | | | | | NS (0,1072) |
| Tacking to teeth | B | A | A | A | *** |
| Crumbleness | | | | | NS (0,0809) |
| Mint flavour | | | | | NS (0,0650) |
| Elasticity | A | C | B | B | ***/** |
| Smoothness | | | | | NS |
| Juiciness | | | | | NS |
| Cooling | | | | | NS |
| Sweetness | A | B | B | B | **/* |
| Softness | | | | | NS (0,0877) |
| Creakiness | A | B | B | B | *** |
| Volume | | | | | NS (0,1841) |

| | | | | | |
|---|---|---|---|---|---|
| ¹* ** *** indicate P< 0.5, < 0.01 and < 0.001, respectively; ²Not significant | | | | | |

### EXAMPLE 11

### Reduced hardness of chewing gums prepared by a one-step process of the invention

It is generally desirable that the masticatory feeling of hardness in a chewing gum body is not too high. It was therefore decided to test the effect on hardness of the final chewing gum prepared using the present one-step process using chewing gum of the same composition, but prepared using a conventional two-step process as references.

The following chewing gums were included in this testing: (i) the chewing gum of Example 2 herein (BDP2, premelted) and (ii) the chewing gum of Example 6 herein (BDP2, one-step).

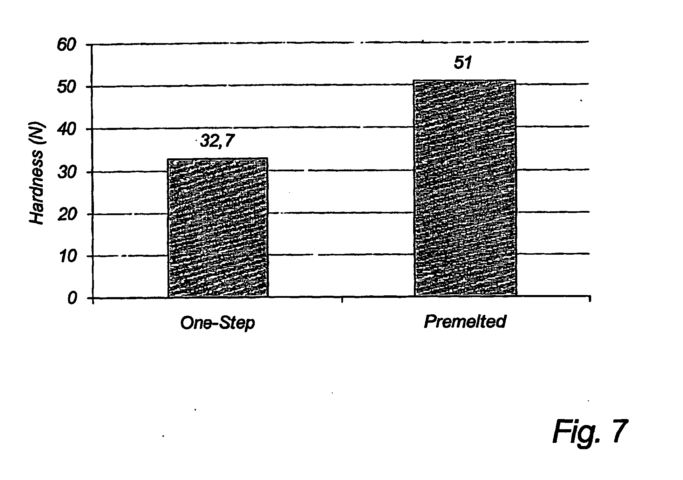

The hardness of the test samples were tested by a compression load test using an Instron instrument with a 4 mm DIA CYLINDER STAINLESS at a speed of 25 mm/min. using a test distance of 3.5 mm into the chewing gum body. The results (N) are summarised in Figure 7. As it appears, the test chewing gum sample prepared using the one-step process of the present invention showed substantially reduced hardness over the conventionally produced chewing gums.

### EXAMPLE 12

In this experiment, the rheological properties (storage modulus, G') was determined using a rheometer, type AR1000 from AT Instruments for the chewing gums made in Example 6 (with degradable gum base polymer BDP2, one-step), Example 2 (with pre-melted degradable gum base polymer BDP2, i.e. a two-step process) and a chewing gum comprising non-degradable gum base polymer B and made by a conventional two-step process wherein the gum base is pre-melted prior to admixing with chewing gum additives. The oscillation measurement is performed at a stress within the linear viscoelastic region and a temperature of 70 °C with a parallel plate system (d=2,0 cm, hatched).

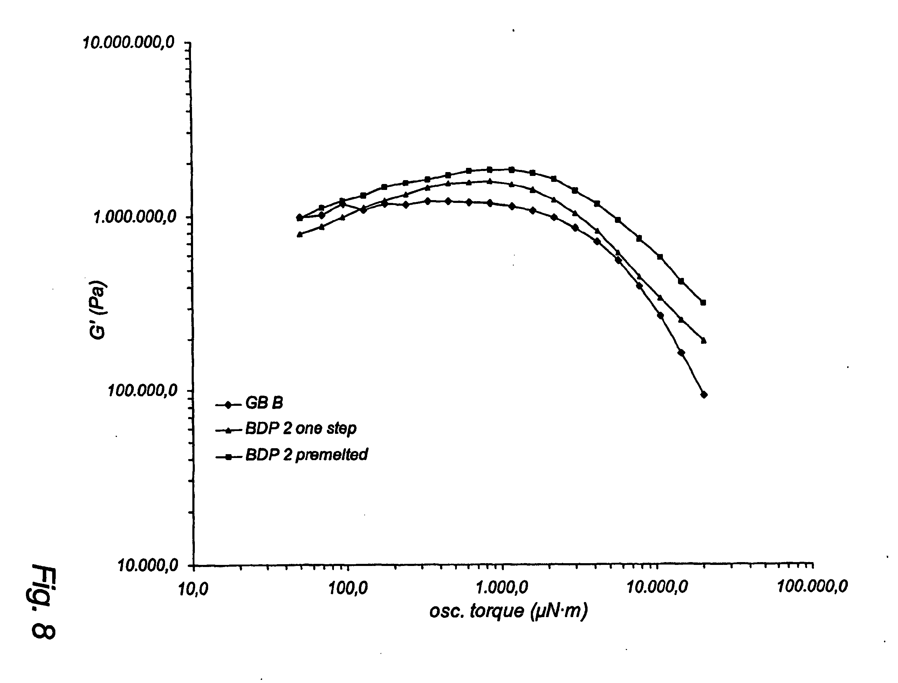

The results are summarised in Fig. 8 and, as it appears, the elasticity of the chewing gum made in accordance with the present invention was improved as compared to the chewing gum made by using pre-melted gum bases and it had elasticity characteristics which were closer to those of the chewing gum made from a non degradable polymer than the one made by using a degradable polymer.

### EXAMPLE 13

### Preparation of chewing gum with peppermint taste using conventional gum base formulations applying a one-step mixing process wherein all of the gum base and chewing gum components are loaded separately to the mixing apparatus

In this Example, different conventional gum base formulations was used to make one step chewing gum formulations. Each of these gum base components was added separately to the mixing apparatus used in the previous Examples. The composition and mixing conditions were as listed in the below Table 13:

**Table 13. Composition of chewing gum with conventional gum base components and one-step mixing conditions.**

| **ONE-STEP PROCESS** | **CG 1-1010** | **CG 2-1008** | **CG 3-1009** | **CG 4-1011** | **CG 5-1542** | **CG 6-1541** |
|---|---|---|---|---|---|---|
| Parts (weight) | % | % | % | % | % | % |
| Elastomer | 8 | 6 | 3 | 11 | 5 | 7 |
| Elastomer plastisizer | 16 | 11 | 17 | 17 | 20 | 11 |
| Softener | 10 | 8 | 9 | 12 | 8 | 10 |
| Filler | 6 | 15 | 11 | - | 7 | 12 |
| Sorbitol powder | 45,6 | 45,6 | 45,6 | 45,6 | 45,6 | 45,6 |
| Lycasin | 6 | 6 | 6 | 6 | 6 | 6 |
| Peppermint oil | 1.5 | 1.5 | 1.5 | 1.5 | 1.5 | 1.5 |
| Menthol (crystal) | 0.5 | 0.5 | 0.5 | 0-5 | 0.5 | 0.5 |
| Aspartame | 0.2 | 0.2 | 0.2 | 0.2 | 0.2 | 0.2 |
| Acesulfame | 0.2 | 0.2 | 0.2 | 0.2 | 0.2 | 0.2 |
| Xylitol | 6 | 6 | 6 | 6 | 6 | 6 |
| ONE STEP | 55 | 55 | 45 | 40 | 43 | 53 |
| Total Proces time (min.) | | | | | | |
| ONE STEP | 48 | 48 | 54 | 49 | 45 | 40 |
| Proces Temperature (°C) | | | | | | |

For comparison the same chewing gum formulations was prepared using conventional two step mixing process. The composition and mixing conditions were as listed in the below Table 14:

**Table 14. Composition of gum base and chewing gum using conventional two-step process.**

| **CONVENTIONAL GUM BASE PROCESS** | **GB 1-1004** | **GB 2-1000** | **GB 3-1002** | **GB 4-1006** | **GB 5-1029** | **GB 6-1028** |
|---|---|---|---|---|---|---|
| Parts (weight) | % | % | % | % | % | % |
| Elastomer | 20 | 15 | 7 | 26 | 16 | 11 |
| Elastomer plastisizer | 40 | 28 | 43 | 44 | 28 | 50 |
| Softener | 25 | 20 | 24 | 30 | 25 | 17 |
| Filler | 15 | 37 | 26 | - | 31 | 22 |
| Proces Temperature (°C) | 120 | 120 | 120 | 120 | 130 | 130 |
| Proces time (min.) | 90 | 90 | 90 | 120 | 55 | 50 |
| **CONVENTIONAL CHEWING GUM PROCESS** | **CG 7-1016** | **CG 8-1012** | **CG 9-1014** | **CG 10-1018** | **CG 11-1539** | **CG 12-1538** |
| Parts (weight) | % | % | % | % | % | % |
| Gum base (GB 1-6) | 40 (GB 1) | 40 (GB 2) | 40 (GB 3) | 40 (GB 4) | 40 (GB 5) | 40 (GB 6) |
| Sorbitol powder | 45,6 | 45,6 | 45,6 | 45,6 | 45,6 | 45,6 |
| Lycasin | 6 | 6 | 6 | 6 | 6 | 6 |
| Peppermint oil | 1.5 | 1.5 | 1.5 | 1.5 | 1.5 | 1.5 |
| Menthol (crystal) | 0.5 | 0.5 | 0.5 | 0.5 | 0.5 | 0.5 |
| Aspartame | 0.2 | 0.2 | 0.2 | 0.2 | 0.2 | 0.2 |
| Acesulfame | 02 | 0.2 | 0.2 | 0.2 | 0.2 | 0.2 |
| Xylitol | 6 | 6 | 6 | 6 | 6 | 6 |
| Proces Temperature (°C) | 25 | 25 | 25 | 25 | 25 | 25 |
| Proces time (min.) | 50 | 50 | 50 | 50 | 50 | 50 |

| Two step | | | | | | |
|---|---|---|---|---|---|---|
| Total Proces time (min.) | 115 | 115 | 115 | 145 | 75 | 75 |

| Two step | | | | | | |
|---|---|---|---|---|---|---|
| Proces Temperature (°C) | 120 (I) | 120 (I) | 120 (I) | 120 (I) | 130 (I) | 130 (I) |
| | 50 (II) | 50 (II) | 50 (II) | 50 (II) | 51 (II) | 40 (II) |

| | | | | | | |
|---|---|---|---|---|---|---|
| I: Conventional gum base process, II: conventional chewing gum process | | | | | | |

Also with different conventional gum base formulations added separately it was possible to obtain the final chewing gum using a one-step mixing process with significant reduced process time and without reaching a higher temperature as in the second step of a conventional two-step processes.

### EXAMPLE 14

In this experiment, the rheological properties (storage modulus, G') was determined using a rheometer, type AR1000 from AT Instruments for the chewing gums made in Example 13. The oscillation measurement is performed at a stress within the linear viscoelastic region and a temperature of 70 °C with a parallel plate system (d=2,0 cm, hatched).

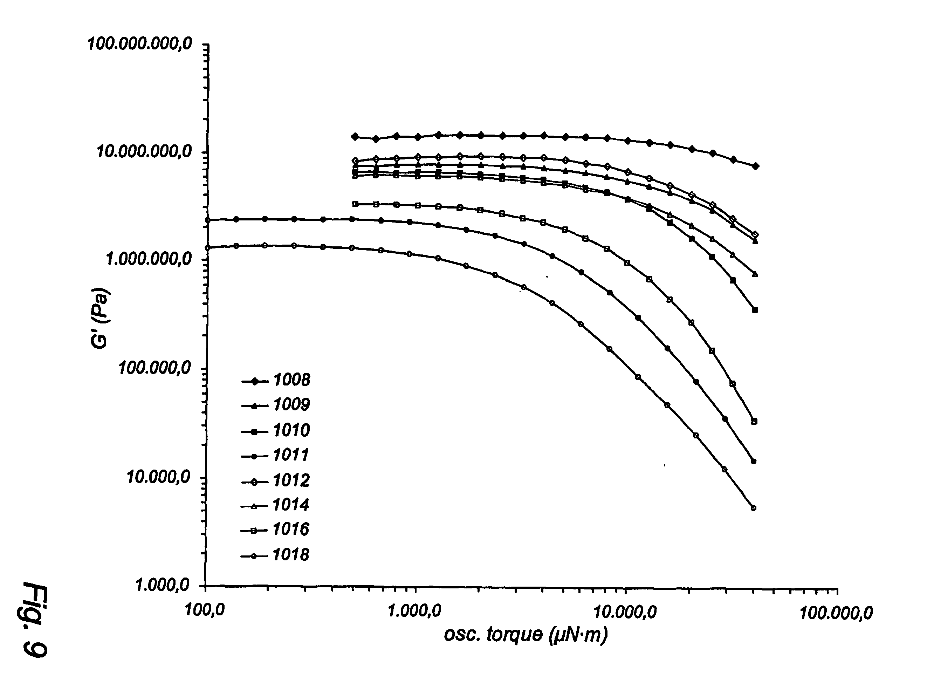

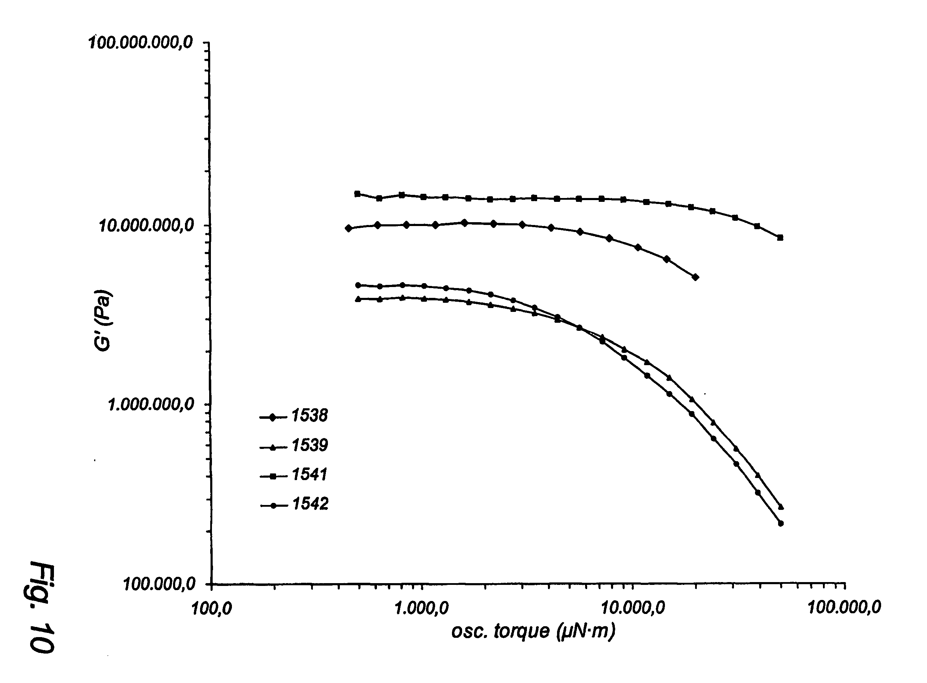

The results are summarised in Fig. 9 and Fig. 10, as it appears, the rheological properties of the chewing gum made in accordance with the present invention are similar as compared to the chewing gum made by conventional two-step process.

### EXAMPLE 15

### Sensory profile analyses of test chewing gum

The following chewing gums were tested by a sensory panel of 5 panellists extensively trained in sensory testings of chewing gum: the chewing gum of Example 13, i.e. a chewing gum made in a one-step process designated herein as CG 1, and the same chewing gum formulation prepared in a conventional two-step process designated as CG 7.

The samples of chewing gum was tested under same conditions described in Example 10.

The following standard parameters were assessed: Flavour impact, flavour intensity, peppermint, tacking to teeth, initial softness, volume, creakiness, softness, sweetness, cooling effect and elasticity.

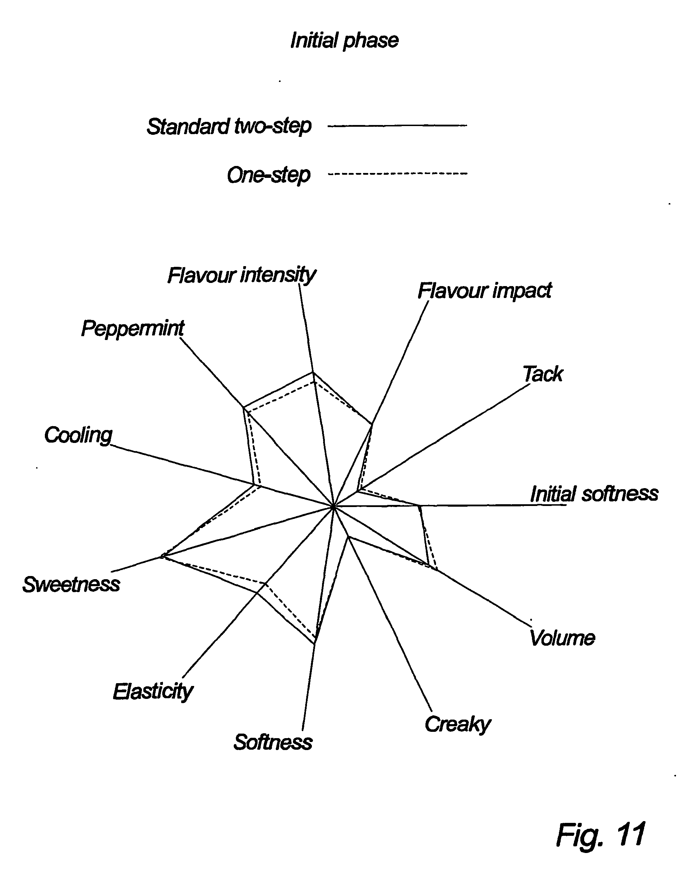

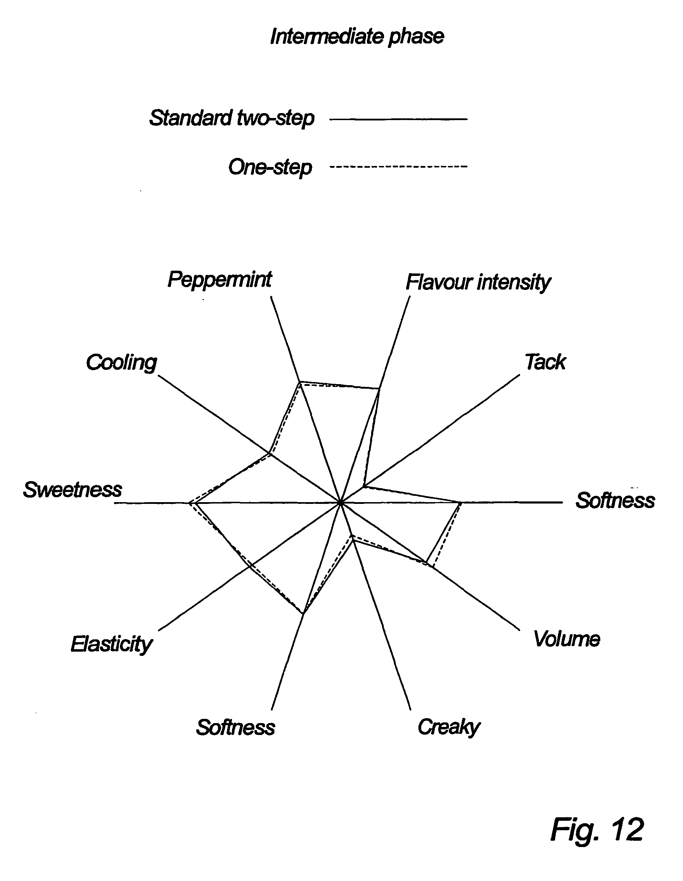

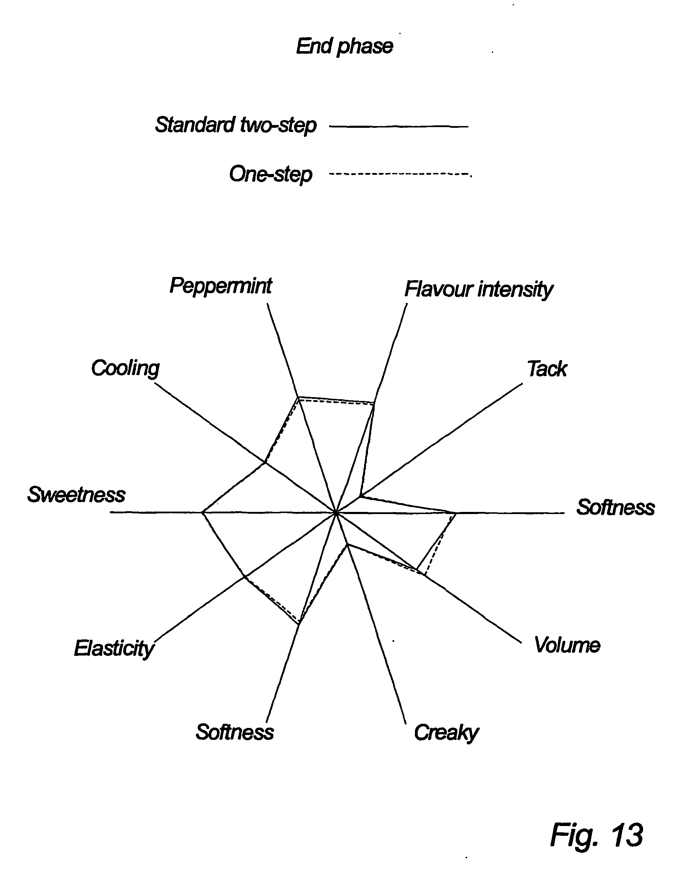

The major differences between test chewing gums in all three testing phases (figures 11,12 and 13) were the following:

Chewing gum prepared using a one-step according to the invention had a significantly larger volume than corresponding chewing gum prepared in a conventional two-step process.

It can therefore be concluded that the use of a one-step chewing gum process without a separate gum base preparation prior to admixture with the chewing gum additives results in final chewing gum products that, relative to chewing gum processed in two separate mixing steps, are superior with regard to volume.

### EXAMPLE 16

### Release of nicotine from conventional medical chewing gum formulation using a one-step mixing process according to the invention

In this Example, conventional medical gum base formulation was used to make one step chewing gum formulation with 2 mg nicotine. Each of these gum base components was added separately to the mixing apparatus used in the previous examples. For comparison a conventional 2 step process was made with same formulation.
The release of nicotine was measured by in vivo and in vitro analysis.

In vivo release of chewing gums are evaluated according to following scale:

| Level | Descripiton | Level of nicotine |
|---|---|---|
| 1 | good | nothing |
| 2 | good, but | very weak |
| 3 | acceptable | "perceptible"- tolerably |
| 4 | unacceptable | strong, unpleasant, difficult to tolerate |
| 5 | totally unacceptable | to strong, -cannot tolerate |

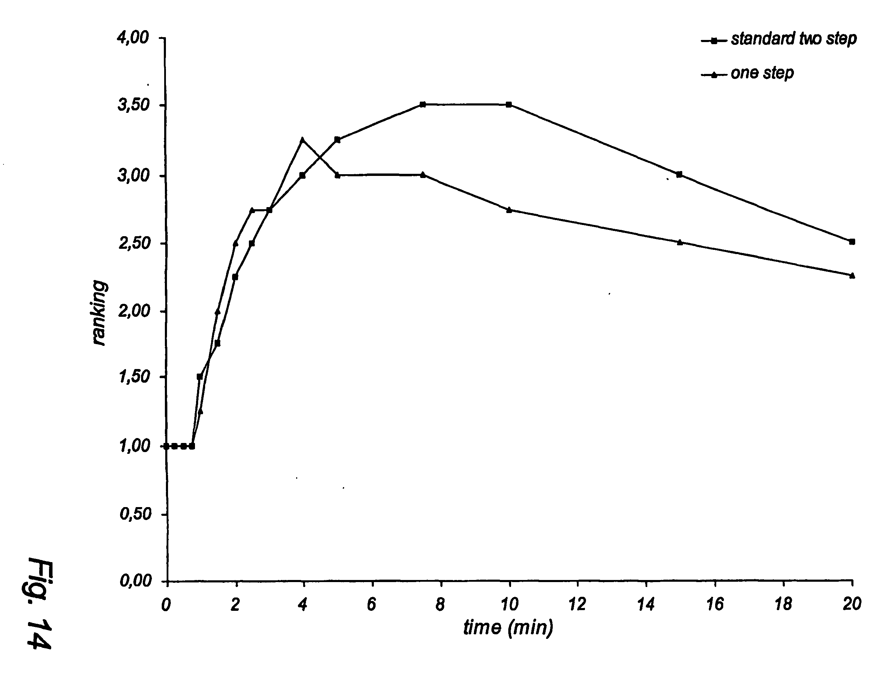

Figure 14 shows the release of nicotine measured in vivo and, as it appears the release of nicotine of the chewing gum made in accordance with the present invention was improved, due to a more acceptable level of nicotine, as compared to the chewing gum made by using the conventional two step process.

Release of nicotine was tested by a HPLC method. The chewing gum was chewed for 10 and 20 minutes in a chewing machine before measurements.
The results of measuring nicotine release in vitro were as listed in the below Table 15:

**Table 15. In vitro release of nicotine.**

| Chewing gum 2 mg nicotine mint | Release nicotine after 10 min chewing | Release nicotine after 20 min chewing |
|---|---|---|
| Conventional 2 step process | 66,28 | 84,73 |
| | 62,88 | 84,21 |
| | 68,45 | 83,58 |
| Average | 65,87 | 84,18 |
| One step process | 56,23 | 81,78 |
| | 61,82 | 80,58 |
| | 57,08 | 81,72 |
| Average | 58,38 | 81,36 |
| % difference | 11 % | 3% |

The release results obtained from the in vitro analysis are confirming the in vivo evaluation obtained in the above Example.

## Claims

1. A process for preparing a chewing gum, the process comprising charging, in an appropriate order, all of the gum base components and all of the chewing gum additives into a mixing apparatus and operating the apparatus at atmospheric pressure to obtain the chewing gum, subject to the limitation that the gum base does not contain a vinyl polyester as the sole functional chewing gum polymer and that the temperature in the chewing gum mixture during operation of the mixing apparatus does not exceed 60°C.

2. A process according to claim 1, wherein the temperature in the chewing gum mixture during operation of the mixing apparatus does not exceed 48°-55°C.

3. A process according to claim 1 or 2 wherein at least one component of the gum base is an environmentally degradable or biodegradable polymer.

4. A process according to claim 3 wherein all of the polymeric components of the gum base are environmentally degradable or biodegradable polymers,

5. A process according to claim 3 or 4 wherein the environmentally degradable or biodegradable polymer is selected from the group consisting of polyesters, polycarbonates, polyester amides, polypeptides and proteins.

6. A process according to claim 1 or 2 wherein at least one component of the gum base is a non-degradable polymer.

7. A process according to claim 6 wherein all of the polymeric components of the gum base are non-degradable polymers.

8. A process according to claim 6 or 7 wherein the non-degradable polymers are selected from the group consisting of polyisobutylene, isobutylene-isoprene copolymer, styrene-butadiene copolymer, polyvinyl acetate (PVA), polyisoprene, polyethylene and vinyl acetate-vinyl laurate copolymer.

9. A process according to any of claims 1-8, which is a batch mixing process.

10. A process according to claim 9 wherein the mixing apparatus is selected from the group consisting of a sigma blade mixer and an extruder.

11. A process according to any of claims 1-8, which is a continuous process.

12. A process according to any of claims 1-11 where the resulting chewing gum is formed into pieces and said pieces are coated.

13. A process according to claim 12 wherein the pieces prior to coating are precoated with at least one layer of a moisture impermeable material.

14. A process according to any of claims 1-13 wherein the gum base components and/or the chewing gum additives include a pharmaceutically or biologically active substance.

15. A process according to claim 14 wherein the pharmaceutically or biologically active substance is nicotine.

## Patentansprüche

1. Verfahren zur Herstellung eines Kaugummis, wobei das Verfahren umfasst, dass man in geeigneter Reihenfolge alle Gummibasis-Komponenten und alle Kaugummi-Zusätze in eine Mischvorrichtung einführt und die Vorrichtung bei Atmosphärendruck betreibt, um den Kaugummi zu erhalten, mit der Maßgabe, dass die Gummibasis keinen Vinylpolyester als alleiniges funktionelles Kaugummi-Polymer enthält und dass die Temperatur in der Kaugummi-Mischung während des Betriebs der Mischvorrichtung 60°C nicht überschreitet.

2. Verfahren nach Anspruch 1, in dem die Temperatur in der Kaugummi-Mischung während des Betriebs der Mischvorrichtung 48°-55°C nicht überschreitet.

3. Verfahren nach Anspruch 1 oder 2, in dem mindestens eine Komponente der Gummibasis ein durch die Umgebung abbaubares oder bioabbaubares Polymer ist.

4. Verfahren nach Anspruch 3, in dem alle Polymer-Komponenten der Gummibasis durch die Umgebung abbaubare oder bioabbaubare Polymere sind.

5. Verfahren nach Anspruch 3 oder 4, in dem das durch die Umgebung abbaubare oder bioabbaubare Polymer ausgewählt ist aus der Gruppe bestehend aus Polyestern, Polycarbonaten, Polyesteramiden, Polypeptiden und Proteinen.

6. Verfahren nach Anspruch 1 oder 2, in dem mindestens eine Komponente der Gummibasis ein nicht abbaubares Polymer ist.

7. Verfahren nach Anspruch 6, in dem alle Polymerkomponenten der Gummibasis nicht abbaubare Polymere sind.

8. Verfahren nach Anspruch 6 oder 7, in dem die nicht abbaubaren Polymere ausgewählt sind aus der Gruppe bestehend aus Polyisobutylen, Isobutylen-Isopren-Copolymer, Styrol-Butadien-Copolymer, Polyvinylacetat (PVA), Polyisopren, Polyethylen und Vinylacetat-Vinyllaurat-Copolymer.

9. Verfahren nach irgendeinem der Ansprüche 1 - 8, bei dem es sich um ein Chargen-Mischverfahren handelt.

10. Verfahren nach Anspruch 9, in dem die Mischvorrichtung ausgewählt ist aus der Gruppe bestehend aus einem Sigma-Blattrührer und einem Extruder.

11. Verfahren nach irgendeinem der Ansprüche 1 - 8, bei dem es sich um ein kontinuierliches Verfahren handelt.

12. Verfahren nach irgendeinem der Ansprüche 1 - 11, in dem der resultierende Kaugummi zu Stücken geformt wird und die Stücke beschichtet werden.

13. Verfahren nach Anspruch 12, bei dem die Stücke vor dem Beschichten mit mindestens einer Schicht aus einem feuchtigkeitsundurchlässigen Material beschichtet werden.

14. Verfahren nach irgendeinem der Ansprüche 1 13, in dem die Gummibasis-Komponenten und/oder die Kaugummi-Zusätze eine pharmazeutisch oder biologisch aktive Substanz einschließen.

15. Verfahren nach Anspruch 14, in dem die pharmazeutisch oder biologisch aktive Substanz Nikotin ist.

## Revendications

1. Procédé de préparation d'un chewing-gum, le procédé comprenant la charge, dans un ordre approprié, de tous les composants de la préparation pour gomme et de tous les additifs de chewing-gum dans un appareil de mélangeage et l'utilisation de l'appareil à la pression atmosphérique pour obtenir le chewing-gum, sous réserve de la limitation selon laquelle la préparation pour gomme ne contient pas un polyester de vinyle en tant que seul polymère de chewing-gum fonctionnel et que la température dans le mélange de chewing-gum durant le fonctionnement de l'appareil de mélangeage ne dépasse pas 60 °C.

2. Procédé selon la revendication 1, dans lequel la température dans le mélange de chewing-gum durant le fonctionnement de l'appareil de mélangeage ne dépasse pas 48 à 55 °C.

3. Procédé selon la revendication 1 ou 2, dans lequel au moins un composant de la préparation pour gomme est un polymère biodégradable ou dégradable de manière environnementale.

4. Procédé selon la revendication 3, dans lequel tous les composants polymères de la préparation pour gomme sont des polymères biodégradables ou dégradables dans l'environnement.

5. Procédé selon la revendication 3 ou 4, dans lequel le polymère biodégradable ou dégradable dans l'environnement est choisi dans le groupe consistant en des polyesters, des polycarbonates, des polyesters-amides, des polypeptides et des protéines.

6. Procédé selon la revendication 1 ou 2, dans lequel au moins un composant de la préparation pour gomme est un polymère non dégradable.

7. Procédé selon la revendication 6, dans lequel tous les composants polymères de la préparation pour gomme sont des polymères non dégradables.

8. Procédé selon la revendication 6 ou 7, dans lequel les polymères non dégradables sont choisis dans le groupe consistant en un polyisobutylène, un copolymère isobutylène-isoprène, un copolymère styrène-butadiène, un acétate de polyvinyle (PVA), un polyisoprène, un polyéthylène et un copolymère acétate de vinyle-laurate de vinyle.

9. Procédé selon l'une quelconque des revendications 1 à 8, lequel est un procédé de mélangeage discontinu.

10. Procédé selon la revendication 9, dans lequel l'appareil de mélangeage est choisi dans le groupe consistant en un mélangeur à pales sigma et une extrudeuse.

11. Procédé selon l'une quelconque des revendications 1 à 8, lequel est un procédé continu.

12. Procédé selon l'une quelconque des revendications 1 à 11, dans lequel le chewing-gum résultant est mis sous forme de morceaux et lesdits morceaux sont enrobés.

13. Procédé selon la revendication 12, dans lequel les morceaux avant l'enrobage sont pré-enrobés avec au moins une couche d'une substance imperméable à l'humidité.

14. Procédé selon l'une quelconque des revendications 1 à 13, dans lequel les composants de la préparation pour gomme et/ou les additifs du chewing-gum incluent une substance pharmaceutiquement ou biologiquement active.

15. Procédé selon la revendication 14, dans lequel la substance pharmaceutiquement ou biologiquement active est la nicotine.
